# EUROPEAN PATENT SPECIFICATION

(11) **EP 1 534 207 B1**
(45) Date of publication and mention of the grant of the patent: **04.04.2007**
(21) Application number: 02739011.1
(22) Date of filing: 27.05.2002
(51) Int. Cl.: A61F 13/56

(54) **ABSORBENT PRODUCT SUCH AS A DIAPER, AN INCONTINENCE PAD OR THE LIKE**
SAUGFÄHIGES PRODUKT, Z.B. WINDEL, INKONTINENZEINLAGE ODER DERGLEICHEN
PRODUIT ABSORBANT TEL QU'UNE COUCHE, UNE SERVIETTE POUR INCONTINENCE OU UN ARTICLE SIMILAIRE

(30) Priority: 07.06.2001 SE 0102016
(43) Date of publication of application: 01.06.2005
(73) Proprietor: SCA Hygiene Products AB, 405 03 Göteborg (SE)
(72) Inventor: LARSSON, Björn, S-427 00 Billdal (SE); HOFFBACK, Britt-Louise, S-412 69 Göteborg (SE); NURMI, Hannele, S-471 42 Rönnäng (SE)
(74) Representative: Romare, Laila Anette
(86) International application number: PCT/SE2002/001003
(87) International publication number: WO 2002/098337

(56) References cited:
- EP-A2- 0 941 728
- WO-A1-98/27921
- US-B1- 6 235 011

## Description

### TECHNICAL FIELD

The present invention relates to an absorbent product, such as a diaper, an incontinence pad or the like, comprising a liquid-permeable surface material, a liquid-impermeable backing material and an absorption body enclosed therebetween. The product also has a front portion, a rear portion and a crotch portion located between the front and rear portions.

### BACKGROUND ART

Conventional absorbent products of this type usually have an attachment system for securing the absorbent product around a wearer. The attachment system may consist of, for example, a first and a second part which interact, for example an attachment flap (tab), and a receiving part (landing zone) for the tab. The tab can be provided with glue or a similar adhesive which is attached to the landing zone which then often consists of a strip of plastic material, for example polypropylene. Alternatively, the tab is designed with a first part and the landing zone with a second part of a touch and close (hook and loop) attachment system. Usually, the tab is provided with the male part or hook part of such a system, and the landing zone with the female part or loop part of the system. The landing zone is normally attached to the front portion of the absorbent product in an area along the front end edge of the product so that it extends in the transverse direction of the product along a considerable part of the width of the product in the waist area. In this way, the tab can interact with the landing zone along the entire length of the landing zone in order that as good an adjustment as possible of the product to the wearer is obtained. Examples of this type of conventional hook and loop attachment system are found in EP 0 276 970 B1, EP 0 324 578 B1 and EP 0 491 347.

US 6,235,011 discloses an absorbent article comprising a central inactive area and two active areas arranged one on each side of the central inactive area.

One problem with this type of receiving strip of the touch and close type is that they are expensive to manufacture. It is not unusual for the cost of the receiving strip to account for up to 10% of the total material cost of the product. Furthermore, as it is attached to the product, the landing zone causes the area where the strip is connected to the product to be stiffer in relation to surrounding areas, which can lead to the product chafing against the body of the wearer.

### BRIEF DESCRIPTION OF THE INVENTION

The object of the present invention is to remedy the abovementioned problems and to construct an absorbent product with a receiving strip which has a relatively low cost as far as material and manufacture are concerned and which at the same time makes the product fit stably on the wearer and also feels soft and pleasant against the body of the wearer of the product.

An absorbent product according to the present invention is characterized in that it consists of a diaper, an incontinence pad or the like, which product comprises a liquid-permeable surface layer, a liquid-impermeable backing material and an absorption body enclosed between the surface layer and the backing material. The product has a front portion, a rear portion and a crotch portion located between the front and rear portions, and also an attachment system for securing the absorbent product on a wearer, the attachment system comprising at least one tab for detachable interaction with a landing zone arranged on the front or rear portion of the product. The landing zone is designed as a strip with two long sides and two short sides which join the two long sides together, and also comprises a central inactive area arranged centrally in the transverse direction on the landing zone, and two active areas arranged one on each side of the central inactive area so that the active areas extend in the transverse direction from the central inactive area to the short sides of the landing zone. The landing tone is further characterized in claim 1.

### DESCRIPTION OF THE INVENTION

By means of the present invention, an absorbent product of the type referred to in the introduction has been produced, which product essentially eliminates the problems mentioned previously.

The absorbent product consists of a diaper or an incontinence pad comprising a liquid-permeable surface layer, a liquid-impermeable backing material and an absorption body enclosed therebetween. The diaper is intended to surround the lower part of the trunk of the wearer like a pair of absorbent pants. It has a front portion intended to face forwards on the wearer during use, a rear portion intended to face backwards on the wearer during use, and a narrower crotch portion located between the front and rear portions, which is intended to be arranged in the crotch of the wearer between the legs of the latter.

The front or rear portion of the absorbent product is provided with at least one attachment flap or tab for securing the absorbent product around the wearer. The tab is intended to interconnect the rear and front portions of the absorbent product by virtue of an attachment element arranged on the tab being attached to a receiving part or landing zone for the attachment element arranged on the front or rear portion of the product. The product is preferably provided with two tabs arranged on the rear side panels of the product, one tab on each rear side panel, and a landing zone on the front portion of the product.

The landing zone is suitably designed as a strip with two short sides and two long sides. The long sides extend parallel or essentially parallel to the front end edge of the diaper along a relatively large part of the front end edge, and the short sides join the two long sides together. The landing zone consists of a material which is adapted to interact with the attachment element. The landing zone preferably consists of the female part (loop part) of a touch and close tape, and the attachment element of the male part (hook part) of the touch and close tape. The female and male parts of the touch and close tape are usually in the form of a support means and, arranged on the support means, a material which constitutes either eyes or loops or, respectively, hooks.

When an absorbent product is correctly adapted to the wearer, that is to say when the wearer is wearing a product of the correct size, it should be designed in such a manner that it fits stably around the body of the wearer. In order that the product is secured stably, the attachment elements of the tabs should attach to the landing zone so that the attachment system interacts in the most suitable manner with the other parts of the product, which means that the attachment elements are to attach to those parts of the landing zone lying out towards the short sides of the landing zone. Use is therefore not made of the central part of the landing zone for attachment.

The landing zone according to the invention therefore comprises a support means which is designed as a strip which corresponds to the extent of the landing zone. On the support means, attachment means, preferably in the form of loop elements, are arranged on those parts of the support means which are located in the areas around the short sides, so that an inactive area of only support material is formed between two active areas which are provided with attachment means. In this way, the landing zone can be divided into two active areas and an intermediate central inactive area. As the attachment means are not arranged on the whole landing zone, it is cheaper and simpler to manufacture, at the same time as the central inactive part is flexible and relatively soft. Another advantage of a landing zone of this type is that any patterns, colours or printing on the support means are more clearly visible if it is free of attachment means.

### BRIEF DESCRIPTION OF THE FIGURES

The invention will be described in greater detail below with reference to the embodiments shown in the drawings, in which
Figure 1 shows a diaper according to the invention seen from above;
Figure 2 shows a section along the line II-II through the diaper in Figure 1;
Figure 3 shows a view of a diaper;
Figure 4 shows a landing zone;
Figure 5 shows a section along the line V-V through the landing zone in Figure 4;
Figures 6a and 6b shows a landing zone according to the invention;
Figure 7 shows a further illustrative embodiment of a landing zone, and
Figure 8 shows a section along the line VIII-VIII through the landing zone in Figure 7.

### DESCRIPTION OF FIGURES AND EMBODIMENTS

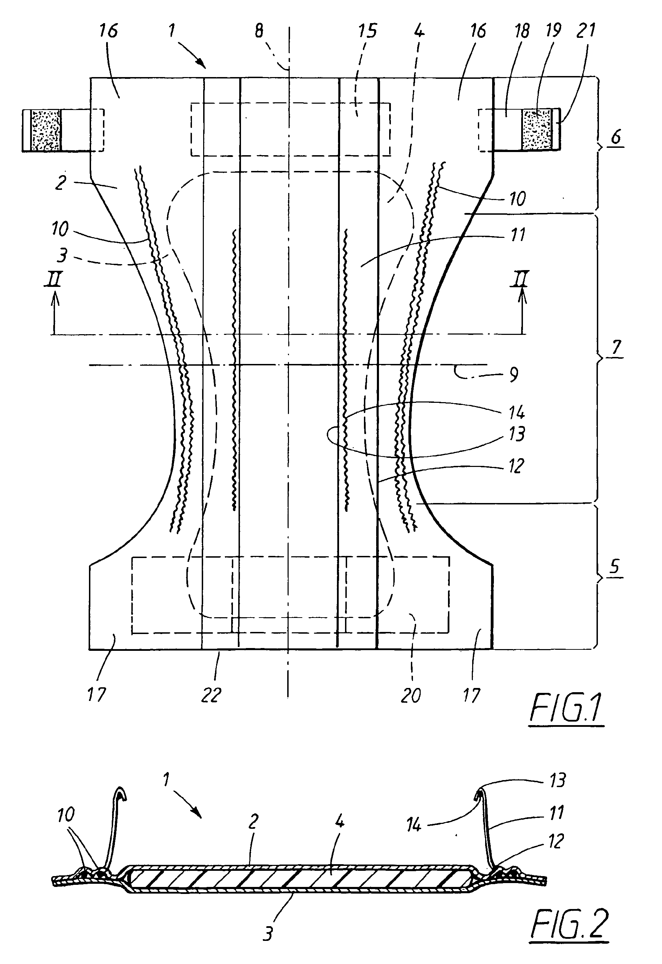

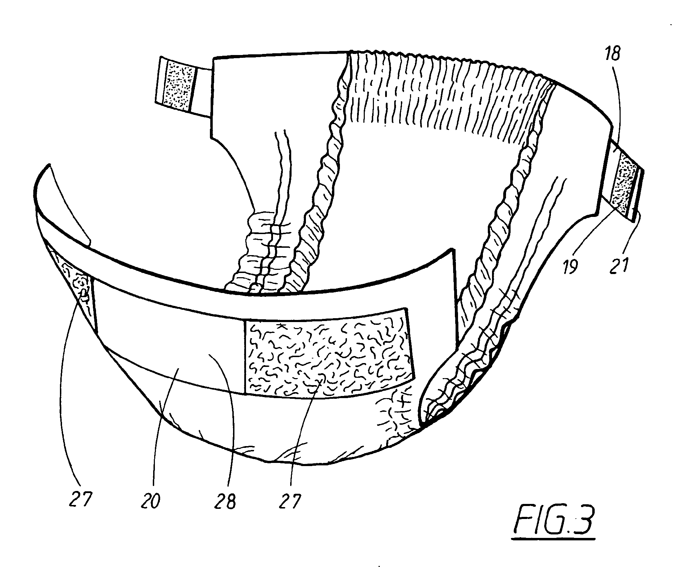

The absorbent product shown in Figures 1-3 consists of a diaper or an incontinence pad 1 comprising a liquid-permeable surface layer 2, a liquid-impermeable backing material 3 and an absorption body 4 enclosed therebetween.

The liquid-permeable surface layer 2 preferably consists of a material which has properties such as dryness and softness during use of the absorbent product, as this layer makes contact with the body of the wearer. It is desirable that the layer has a soft and textile-like surface which remains dry even if wetted repeatedly. The surface layer can consist of, for example, a non-woven material with a soft and smooth surface such as, for example, a spunbond made of polypropylene fibres, a meltblown material or a bonded carded fibre material. In order to keep the surface next to the skin of the wearer dry, a hydrophobic non-woven material can be used, which is perforated so that openings are formed in the material which are larger than the cavities between the fibres in the material. In this way, liquid can be conducted through the openings made in the surface layer down to the underlying absorption body. Other examples of material for the surface layer are, for example, perforated plastic films such as, for example, perforated polyethylene film.

The liquid-impermeable backing material 3 can consist of a thin film of polyethylene (PE), polypropylene (PP) or another suitable material, a hydrophobed non-woven layer or a laminate of a thin film and a non-woven material. This type of laminate is often used to obtain a soft, textile-like exterior on the backing layer. In order to achieve a more airy and pleasant product, it is also possible to make use of breathable backing layers which prevent liquid escaping from the absorbent product but allow moisture to be ventilated out. These breathable backing layers can consist of single material layers or of laminates of, for example, blown or moulded polyethylene films which are laminated with, for example, a non-woven layer made of spunbond or spunbond-meltblown-spunbond (SMS).

The surface layer 2 and the backing material 3 have a slightly greater extent in the plane than the absorption body 4 and extend outside the edges of the latter. The layers 2 and 3 are interconnected in the projecting portions, for example by gluing or welding using heat or ultrasound. They can also be connected to the absorption body by, for example, glue.

The absorption body 4 can be of any conventional type. The absorption body is usually made up of one or more layers of cellulose fibres, for example cellulose fluff pulp. Other materials which can be used are, for example, absorbent non-woven materials, foamed materials, synthetic fibre materials or peat. In addition to cellulose fibres or other absorbent materials, the absorption body can also contain superabsorbent material, what are known as SAPs (superabsorbent polymers), that is to say material in the form of fibres, particles, granules, film or the like, which has the capacity to absorb liquid corresponding to several times the weight of the superabsorbent material itself. The superabsorbent material binds the liquid and forms a liquid-containing gel. The absorption body can also comprise binders and shape-stabilizing components or the like. Additional absorption layers which improve the absorption properties can also be used, such as various types of liquid-spreading material layers or inserts, what are known as wadding. The absorption body can be treated chemically or physically in order to modify the absorption properties. It is possible, for example, to provide an absorption layer with compressions in order to control the liquid flow in the absorption body. It is also possible to enclose the absorbent layer or the absorbent layers in a covering made of, for example, tissue material. The absorption body usually has an elongate shape in the longitudinal direction and can be, for example, essentially rectangular, T-shaped or hourglass-shaped. In a T-shaped absorption body, the transverse part is intended to face the front portion of the absorbent product during use so that it lies in the area around the abdomen of the wearer during use. An hourglass-shaped absorption body is wider in the front and rear portions than in the crotch portion in order to afford effective liquid take-up at the same time as the design makes it easier for the product to shape itself and close tightly around the wearer.

The diaper is intended to surround the lower part of the trunk of the wearer like a pair of absorbent pants. It has a front portion 5 intended to face forwards on the wearer during use, a rear portion 6 intended to face backwards on the wearer during use, and a narrower crotch portion 7 located between the front and rear portions, which is intended to be arranged in the crotch of the wearer between the legs of the latter.

The diaper is suitably symmetrical with regard to a longitudinal axis 8 running in the longitudinal direction of the diaper. The transverse direction of the diaper is the direction which extends across the product and is parallel or on the whole parallel to a transverse axis 9 which forms a right angle to the longitudinal axis 8.

Those parts on both sides of the crotch portion 7 in the transverse direction which extend outside the absorption body are preferably provided with one or more elastic means 10 which run essentially in the longitudinal direction of the diaper. The elastic means 10 function as leg elastic and serve the purpose of preventing liquid and motions leaking out through the longitudinal side edges of the diaper and in this way form outer liquid barriers. The elastic means can consist of one or more elastic threads or strips which are applied in the stretched state between the backing layer and the surface layer. Alternatively, the elastic can be arranged between the layers in the unstretched state and the two layers instead are then stretched during application. It is also possible to arrange the elastic on the outside of the backing layer or on the inside of the surface layer.

To provide further protection against liquid or motions leaking out, the absorbent product can also be provided, on the side facing the wearer, with inner liquid barriers 11 which are attached close to the longitudinal edges inside the outer liquid barriers. The inner liquid barriers 11 are preferably made from an essentially liquid-impermeable material such as, for example, a hydrophobic non-woven or a plastic film and are designed as a longitudinal web with a first edge 12 which is connected to the absorbent product and a second, free edge 13 which is intended to make contact with the wearer during use of the absorbent product. The second edge is provided with one or more elastic elements 14, preferably an elastic thread, which, in the contracted state, gathers the free edge so that an upright barrier is formed. The inner barrier can be designed as a strip made of a single layer where the free edge is folded down in order to enclose the elastic element so as to prevent direct contact of the elastic thread with the wearer. Alternatively, the barrier can be made from two layers joined together with the elastic thread attached at the edge of the free end between the two layers. In this case, the inner layer of the barrier can consist of an extension of the surface layer, and the outer layer of an essentially liquid-impermeable material, or the inner and outer layers of the barrier can consist of one and the same material strip which is folded around the elastic thread.

The rear and/or front portions of the product can also be provided with waist elastic 15 which consists of elastic means arranged along the front and/or rear end edges in order to enable the product to enclose the waist of the wearer in a soft and flexible manner. The elastic means are suitably attached between the backing material and the surface layer by glue or by welding, for example ultrasonic welding. The elastic means can consist of one or more elastic threads which are applied in the stretched state between the layers and in this way form the waist elastic. Alternatively, the elastic can be arranged between the layers in the unstretched state and the two layers instead are then stretched during application. Another common variant of elastic which is suitable is elastic foamed material consisting of a thin strip of, for example, polyurethane foam which can be arranged between the two layers in the same way as the elastic threads. It is of course also possible to position the elastic means for the waist elastic on the outside of the backing layer or on the inside of the surface layer.

Those parts of the rear portion of the diaper which, in the transverse direction on both sides of the longitudinal axis 8, extend outside the absorption body constitute the rear side panels 16 of the diaper. In the same way, the absorbent product can be provided with front side panels 17. The front or rear side panels of the absorbent product are provided with an attachment system for securing the absorbent product around a wearer. The attachment system consists of at least one attachment flap or tab 18 and a receiving part or landing zone 20 for the tab 18. The tab 18 is intended to interconnect the rear and front portions of the absorbent product by virtue of an attachment element 19 arranged on the tab 18 being attached to the landing zone 20 which is arranged on the front or rear portion of the product. The product is preferably provided with two tabs arranged on the rear side panels, one tab on each rear side panel 16, and a landing zone 20 in the front portion 5 of the product on the side facing away from the wearer during use of the diaper, that is to say on the outside of the backing material.

The tabs 18 are connected to the rear side panels 16 in the areas which lie at the side edge of the side panels, which runs in the longitudinal direction. The connection can be brought about by, for example, glue, tape, heat-sealing or welding at separate points, along lines or over continuous surface areas. The tabs 18 can be attached to the backing material or surface layer of the absorbent product, between the backing material and the surface layer, or be designed so that the tabs are attached so that one part of the tab lies on the outside of the backing material and another part of the tab lies on the inside of the product, that is to say on that side of the surface layer which faces the wearer.

In the present invention, the attachment element 19 preferably consists of a male part of a touch and close (hook and loop) tape. The attachment element 19 is attached to the tab 18 by, for example, glue, tape, by thermal connection or by another suitable attachment means, on that part of the tab 18 which faces away from the rear side panels 16 of the product and on that side of the tab 18 which faces the landing zone 20 during use of the diaper. That part of the tab 18 which is arranged outside the attachment element 19 in the lateral direction constitutes a gripping tab 21 which serves the purpose of making it easier to apply and remove the attachment element to and from the landing zone 20.

The landing zone 20 is suitably designed as an oblong strip which extends essentially parallel to the front end edge 22 of the diaper, that is to say in the transverse direction of the diaper, and consists of a material which is adapted to interact with the attachment element 19. The extent of the landing zone in the transverse direction of the diaper can vary depending on style and size and can therefore extend along the entire front end edge 22 of the diaper or along a part thereof. The landing zone suitably extends along a relatively large part of the front end edge 22 so that it runs out over the front side panels in order that as good an adjustment as possible of the diaper to the wearer can be obtained. The extent of the landing zone 20 in the longitudinal direction of the diaper can also vary depending on how it is to be adapted to the diaper, but is suitably adapted to the extent of the attachment element 19 in the same direction. The landing zone 20 preferably consists of the female part (loop part) of a touch and close tape, and the attachment element 19 of the male part (hook part) of the touch and close tape. It would also be possible to use the hook and loop parts the other way round, but the loop part is more suitable as the landing zone because it does not have the same problem as a hook part which easily catches on clothing and other materials.

The female and male parts of the touch and close tape are usually in the form of a support means and, arranged on the support means, a material which constitutes either loops or eyes or, respectively, hooks.

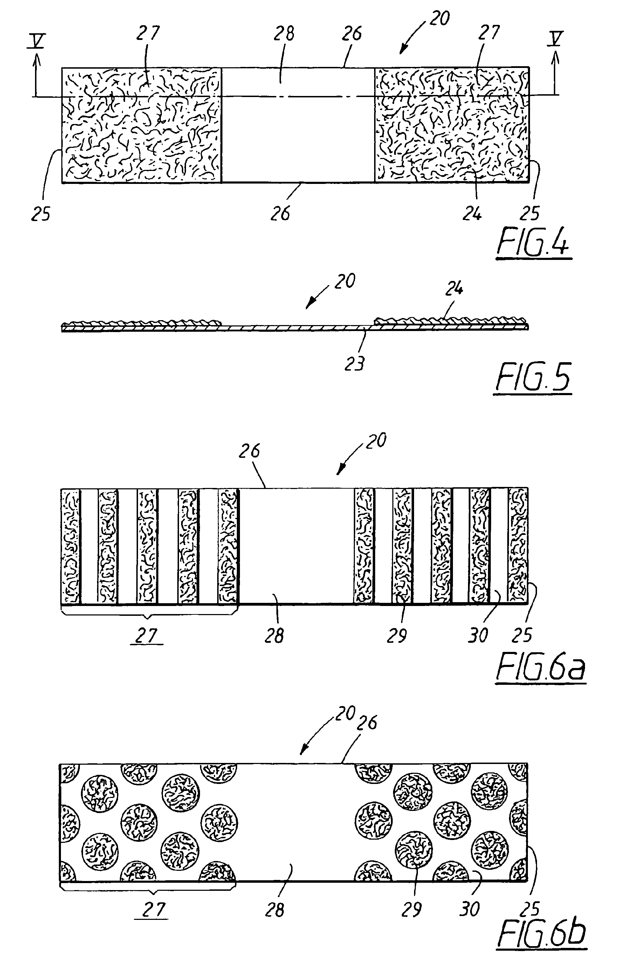

The landing zone 20 will be described in greater detail below. The landing zone 20 consists of a continuous support means 23 which is designed as a strip, which corresponds to the extent of the landing zone, with two long sides 26 and two short sides 25 which join the two long sides together. The long sides 26 are parallel or essentially parallel to the front end edge 22 of the diaper and therefore extend in the transverse direction of the diaper, and the short sides 25 are suitably straight and arranged at right angles to the long sides 26. It is also possible for the short sides 25 to be rounded or to have another shape which is suitable for the diaper to which the landing zone 20 is attached. On the support means 23, attachment means 24, preferably in the form of loop elements, are arranged on only those parts of the support means which, in the transverse direction, are located in the areas around the short sides 25, so that a central inactive area 28 consisting of only the support means 23 is formed between active areas 27 which extend in the transverse direction from the central inactive area 28 to the short sides 25 of the landing zone and consist of the support means 23 and the attachment means 24 (see Figures 4 and 5). In the longitudinal direction of the diaper, both the central inactive area 28 and the two active areas 27 extend along the entire extent of the landing zone. The active areas 27 can therefore interact with the attachment element 19. In the embodiment according to Figures 4 and 5, the attachment means 24 has such an extent that it completely covers the active areas 27 in order that the attachment element 19 is secured as well as possible along the entire extent of the active areas.

The manufacture itself of the landing zone is not described in greater detail in the application because any suitable method of manufacturing touch and close parts with support means and attachment means can be used for the present invention. Suitable materials for the support means 23 and the attachment means 24 may be, for example, polyethylene, polypropylene, polyester, polyamide or another thermoplastic, as well as even biodegradable materials such as, for example, polylactide, polycaprolactone or other polyhydroxyalkanoates and materials, for example polymer materials, manufactured from renewable raw materials such as, for example, maize starch or cellulose. Examples of methods of manufacturing male parts or female parts for attachment elements are described in greater detail in US 5,830,298 A, US 5,611,791 A, US 5,032,122 and US 5,318,741 A, and biodegradable materials in US 5,939,467 A and WO 00/01339 A1. It is a common feature of the attachment elements described and the methods of manufacturing them that the touch and close strip itself is made up of a substrate or support part and that material in the form of hooks or loops is arranged on the substrate. By not arranging hooks or loops on parts of the support means in the manufacturing process, it is possible to manufacture a landing zone according to the invention.

According to the invention, the landing zone 20 is, as described previously, designed with a centrally positioned inactive area 28, and two active areas 27 are arranged one on each side of the central inactive area 28. In this case, the active areas 27 are in turn divided into active part areas 29, and the landing zone is inactive between the active areas owing to either one or a number of inactive part areas 30 (see Figures 6a and 6b). The central inactive area 28 and the inactive part areas 30 consist of the support means 23 in the same way as described previously, and the active part areas of support means 23 and attachment means 24. By virtue of the fact that a smaller part of the surface of the landing zone is provided with attachment means, it is possible to reduce the manufacturing cost further. As the active part areas 29 of the landing zone 20 extend from the short sides 25 in towards the central inactive area 28, good attachment of the attachment element 19 to the landing zone 20 is obtained. It is possible within the scope of the invention to vary the design of the active part areas; they do not have to be designed as oblong or circular areas as shown in Figures 6a and 6b. The different areas can have any shapes which are suitable for achieving secure retention.

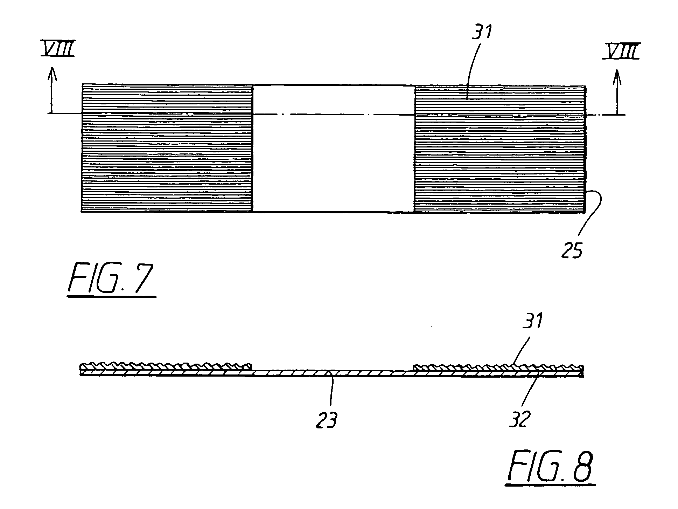

According to another suitable embodiment (see Figures 7 and 8), the landing zone 20 can be designed as a laminate of a non-woven material or a plastic film and continuous fibres 31, what is known as tow. The fibres can be straight, crimped or curled. In this case, the support means 23 consists of the non-woven layer or the plastic film and the attachment means 24 of the continuous fibres. The fibres 31 are fastened to the non-woven layer so that eyes or loops are formed, in which the attachment element 19 can engage. The continuous fibres can be produced separately before they are used in the process for manufacturing the landing zone. The fibres are often supplied in long fibre bundles or packs with a number of fibres arranged side by side. Before the fibres are fastened to the non-woven layer, they should first be separated so that they are arranged lying in a plane essentially parallel side by side so that they can be attached to the non-woven layer in a suitable manner. This can be effected in the manufacturing process by, for example, the fibre bundle being stretched and spread out to form a layer of essentially uniform thickness. Another method may be to introduce a number of fibres arranged side by side in a plane into the manufacturing process. The continuous fibres 31 are then fastened together with the support means 23 in fastening areas 32, suitably along lines, at points or in spots, but the fibres are otherwise not fastened to one another. Fastening is brought about by, for example, ultrasonic welding or other thermal bonding with simultaneous compression. Other possible ways of fastening the fibres to the support means may be by means of binders or mechanical fibre entangling. By virtue of fastening the fibres only to the areas next to the short sides 25 of the support means during manufacture, a landing zone according to the invention is obtained. Suitable materials for the fibres in the tow may be polyethylene, polypropylene, polyamide, polyester, polyvinyl acetate, cellulose acetate, regenerated cellulose fibres or bicomponent fibres.

Examples of handling tow in manufacturing processes are given in, for example, European Patent Application EP 0 937 792 A1.

One or both of the components forming the laminate may also be made from a biodegradable material such as, for example, polylactide, polycaprolactone or other polyhydroxyalkanoates. A landing zone made entirely of a biodegradable material is in this way suitable for use in an absorbent product which can be composted.

The invention is not to be considered as being limited to the embodiments above as these are intended only to explain the invention. The scope of the invention also includes combining characteristics from different embodiments with one another. It is also possible to use this type of landing zone on what are known as belt products. A belt product means a diaper or incontinence pad with an integral belt or belt segments for securing the diaper or incontinence pad on the wearer. The belts may also be detachable from the diaper or the incontinence pad

## Claims

1. Absorbent product, such as a diaper, an incontinence pad or the like, which product comprises a liquid-permeable surface layer (2), a liquid-impermeable backing material (3) and an absorption body (4) enclosed between the surface layer (2) and the backing material (3), the product having a front portion (5), a rear portion (6) and a crotch portion (7) located between the front and rear portions, and an attachment system for securing the absorbent product on a wearer, the attachment system comprising at least one tab (18) for detachable interaction with a landing zone (20) arranged on the front or rear portion of the product, the landing zone being designed as a strip with two long sides (26) and two short sides (25) which join the two long sides (26) together, the landing zone (20) comprising a central inactive area (28) arranged centrally in the transverse direction on the landing zone, and two active areas (27) arranged one on each side of the central inactive area (28), **characterized in that** the active areas extend in the transverse direction from the central inactive area (28) to the short sides (25) of the landing zone and **in that** the landing zone (20) consists of a continuous support means (23), and **in that** the central inactive area (28) consists of the support means (23), and **in that** the active areas (27) are divided into active part areas (29) and inactive part areas (30).

2. Absorbent product according to Claim 1, **characterized in that** the active part areas (29) consist of the support means (23) and attachment means (24) arranged on the support means, and **in that** the inactive part areas (30) consist of the support means.

3. Absorbent product according to any one of Claims 1 or 2, **characterized in that** the attachment means (24) consists of loop elements which interact with attachment elements (19) in the form of hook elements arranged on the tab (18).

4. Absorbent product according to any one of claims 1-3, **characterized in that** the landing zone (20) is in the form of a laminate, where the support means (23) consists of a non-woven layer or a plastic film and the attachment means (24) consists of tow (31).

5. Absorbent product according to Claim 4, **characterized in that** the tow (31) is fastened to the non-woven layer or the plastic film so that eyes or loops are formed.

6. Absorbent product according to any one of the preceding claims, **characterized in that** the landing zone is made from a biodegradable material.

7. Absorbent product according to any one of the preceding claims, **characterized in that** the landing zone is made from a material produced from renewable raw materials.

## Patentansprüche

1. Absorbierendes Produkt wie beispielsweise eine Windel, ein Inkontinenzpad und ähnliches, umfassend eine flüssigkeitsdurchlässige Oberflächenlage (2), ein flüssigkeitsundurchlässiges Deckmaterial (3) und einen Absorptionskörper, der zwischen der Oberflächenlage (2) und dem Deckmaterial (3) eingeschlossen ist, wobei das Produkt einen Vorderabschnitt (5), einen Hinterabschnitt (6) und einen Schrittabschnitt (7), der zwischen dem Vorder- und dem Hinterabschnitt angeordnet ist sowie ein Befestigungssystem zum Befestigen des absorbierenden Produkts am Träger aufweist, wobei das Befestigungssystem wenigstens eine Lasche (18) zur lösbaren Wechselwirkung mit einem auf dem Vorder- oder Hinterabschnitt des Produkts angeordneten Aufbringbereich (20) umfasst, wobei der Aufbringbereich als ein Streifen mit zwei langen Seiten (26) und zwei kurzen Seiten (25), die die zwei langen Seiten (26) miteinander verbinden, ausgestaltet ist, einen mittigen inaktiven Bereich (28), der in Querrichtung mittig auf dem Aufbringbereich angeordnet ist und zwei aktive Bereiche (27), die auf jeder Seite des mittigen inaktiven Bereichs (28) angeordnet sind, umfasst, **dadurch gekennzeichnet, dass** sich die aktiven Bereiche in Querrichtung von dem mittigen inaktiven Bereich (28) zu den kurzen Seiten (25) des Aufbringbereichs erstrecken, dass der Aufbringbereich (20) aus einer kontinuierlichen Trägereinrichtung (23) besteht, dass der mittige inaktive Bereich (28) aus der Trägereinrichtung (23) besteht, dass die aktiven Bereiche (27) in aktive Teilbereiche (29) und inaktive Teilbereiche (30) unterteilt sind.

2. Absorbierendes Produkt nach Anspruch 1, **dadurch gekennzeichnet, dass** die aktiven Teilbereiche (29) aus der Trägereinrichtung (23) bestehen und eine Hafteinrichtung (24) auf der Trägereinrichtung angeordnet ist, dass die inaktiven Teilbereiche (30) aus der Trägereinrichtung bestehen.

3. Absorbierendes Produkt nach einem der Ansprüche 1 oder 2, **dadurch gekennzeichnet, dass** die Hafteinrichtung (24) Schlaufenelemente umfasst, die mit Haftelementen (19) in Form von Hakenelementen, die auf der Lasche (18) angeordnet sind, zusammenwirken.

4. Absorbierendes Produkt einem der Ansprüche 1-3, **dadurch gekennzeichnet, dass** der Aufbringbereich (20) in Form eines Laminats vorliegt, wobei die Trägereinrichtung (23) aus einer Vliesstofflage oder einer Kunststofffolie und die Hafteinrichtung (24) aus Garn (31) besteht.

5. Absorbierendes Produkt nach Anspruch 4, **dadurch gekennzeichnet, dass** das Garn (31) derart an der Vliesstofflage oder der Kunststofffolie befestigt wird, dass Ösen oder Schlaufen ausgebildet sind.

6. Absorbierendes Produkt nach einem der vorstehenden Ansprüche, **dadurch gekennzeichnet, dass** der Aufbringbereich aus einem biologisch abbaubaren Material gebildet ist.

7. Absorbierendes Produkt nach einem der vorstehenden Ansprüche, **dadurch gekennzeichnet, dass** der Aufbringbereich aus einem Material gebildet ist, das aus erneuerbaren Rohstoffen hergestellt ist.

## Revendications

1. Produit absorbant, tel qu'une couche, une serviette pour incontinence ou similaire, lequel produit comprend une couche de surface perméable aux liquides (2), un matériau protecteur imperméable aux liquides (3) et un corps d'absorption (4) enserré entre la couche de surface (2) et le matériau protecteur (3), le produit ayant une partie avant (5), une partie arrière (6) et une partie d'entrejambes (7) située entre les parties avant et arrière, et un système d'attache permettant de fixer le produit absorbant sur un utilisateur, le système d'attache comprenant au moins une languette (18) destinée à une interaction détachable avec une zone d'appui (20) agencée sur la partie avant ou la partie arrière du produit, la zone d'appui étant conçue sous la forme d'une bandelette dotée de deux côtés longs (26) et de deux côtés courts (25) qui réunissent ensemble les deux côtés longs (26), la zone d'appui (20) comprenant une zone inactive centrale (28), agencée au centre, dans la direction transversale sur la zone d'appui, et deux zones actives (27) agencées une de chaque côté de la zone inactive centrale (28), **caractérisée en ce que** les zones actives s'étendent dans la direction transversale à partir de la zone inactive centrale (28) jusqu'aux côtés courts (25) de la zone d'appui et **en ce que** la zone d'appui (20) consiste en un moyen de support continu (23), et **en ce que** la zone inactive centrale (28) consiste en le moyen de support (23) et **en ce que** les zones actives (27) sont divisées en des zones de partie active (29) et des zones de partie inactive (30).

2. Produit absorbant selon la revendication 1, **caractérisé en ce que** les zones de partie active (29) consistent en le moyen de support (23) et le moyen d'attache (24) agencé sur le moyen de support, et **en ce que** les zones de partie inactive (30) consistent en le moyen de support.

3. Produit absorbant selon l'une quelconque des revendications 1 ou 2, **caractérisé en ce que** le moyen d'attache (24) consiste en éléments de boucle qui interagissent avec les éléments d'attache (19) sous la forme d'éléments de crochet agencés sur la languette (18).

4. Produit absorbant selon l'une quelconque des revendications 1 à 3, **caractérisé en ce que** la zone d'appui (20) a la forme d'un stratifié, dans lequel le moyen de support (23) consiste en une couche de non tissé ou un film de plastique, et le moyen d'attache (24) consiste en filasse (31).

5. Produit absorbant selon la revendication 4, **caractérisé en ce que** la filasse (31) est fixée sur la couche de non tissé ou le film de plastique, de telle sorte que des oeillets ou des boucles soient formées.

6. Produit absorbant selon l'une quelconque des revendications précédentes, **caractérisé en ce que** la zone d'appui est composée d'un matériau biodégradable.

7. Produit absorbant selon l'une quelconque des revendications précédentes, **caractérisé en ce que** la zone d'appui est composée d'un matériau produit à partir de matières premières renouvelables.
